# EUROPEAN PATENT APPLICATION

(11) **EP 2 818 862 A1**
(43) Date of publication of application: **31.12.2014**
(21) Application number: 14175060.4
(22) Date of filing: 30.06.2014
(51) Int. Cl.: G01N 33/14

(54) **Apparatus and method for light exposure and analysis of fluid products**

(30) Priority: 28.06.2013 IT UD20130091
(71) Applicant: Ever Srl, 31100 Treviso (IT)
(72) Inventor: Branca, Giovanni Enrico, 30026 Portogruaro (VE) (IT)
(74) Representative: Petraz, Gilberto Luigi

(57) **Abstract**

A device to analyze fluid products, configured to subject them to luminous stress, comprises a containing body (11), portable and completely closable, with an internal compartment (16) for housing one or more receptacles (17) containing the fluid products. The containing body (11) is defined by a first part (11a) and by a second part (11b) hinged to each other and delimited by external walls formed by plates (14) made of heat conductive material. The device also comprises a lighting unit (12), inside the internal compartment (16) and provided with sources of light (19) located in contact with one or more of the plates (14), and a power unit (13) associated to the containing body (11). The device to analyze fluid products also comprises, for each of the parts (11a, 11b) of the containing body (11), at least a transparent sheet (22, 23), substantially parallel to one plate (14). Said sources of light (19) are interposed between the plate (14) and a corresponding transparent sheet (22, 23).

## Description

### FIELD OF THE INVENTION

The present invention concerns a device and a method used to analyze fluid products. Here and hereafter in the text, by the term "fluid products" we refer to both liquid and semi-liquid, viscous, pasty or creamy products.

The present invention can be used to analyze, for example but not only, alcoholic or non-alcoholic drinks, for example beer, soft drinks or wine, the latter being white, red or rose, semi-worked products of such drinks such as musts, or other fluid food products, for example oil, vinegar or sauces, or again other non-food products, for example medicines or cosmetics, for example perfumes or detergents, for example used for personal hygiene or for the home.

In particular, the present invention is based on the use of light sources to simulate exposure to light to which said fluid products could be subjected during production, storage, preservation or display at sales points or administration points, and to analyze their stability.

### BACKGROUND OF THE INVENTION

It is known that a plurality of fluid products, both in the field of foods or in other fields, such as for example medicine, cosmetics or others, can contain chemical substances sensitive to light. It is known that for such fluid products therefore, exposure to luminous radiations can determine the alteration of some chemical and physical properties.

It is known, merely by way of example, that some drinks, alcoholic ones for example, in particular wines, can be affected by unwanted chemical and physical changes due to exposure to determinate temperatures and to luminous radiations that can influence the organoleptic quality, even negatively for example.

In order to maintain the quality of a drink, understood as the preservation of its organoleptic properties and aromatic characteristics, a constant control of the production process and a correct preservation downstream of the latter are therefore necessary.

Another example is that of the alteration of the active principle of some light-sensitive medicines.

The correct preservation of a light-sensitive fluid product usually provides to maintain it at low temperatures and protect it from luminous radiations.

In the particular case of wine, this is normally preserved at a temperature in the order of 10°C - 15°C or less.

In some cases, these stratagems may not be adopted, either through lack of awareness on the part of the consumer or for commercial reasons linked to the marketing of the product, in particular at sales points of the final product or where the fluid product is administered to the public.

With the exception of refrigerators of specialized structures, for the most part fluid products are preserved at room temperature, that is, at a temperature considerably higher than the optimal preservation temperature.

Moreover, the positioning of fluid products, especially in supermarkets, shops or restaurants, normally follows the criterion of maximum visibility, exposing the products to artificial light, normally particularly intense, and for long periods of time.

It is known that both natural luminous radiation and also artificial luminous radiation are damaging for the quality of light-sensitive fluid products.

Wine particularly can be affected by radiations astride the spectrum of visible and ultraviolet light and with wavelengths comprised between about 370 nm and about 450 nm. It is known that such wavelengths correspond to the two wavelengths of riboflavin or vitamin B2 absorption. Vitamin B2, that is, the coenzyme of oxidation-reduction involved in the cytochrome bridge, constitutes a system of electro-active and light-sensitive oxidation-reduction. In conditions where oxygen is absent, above all in white wines, reduction reactions of vitamin B2 in its colorless form occur, due to the reduction in the oxidation-reduction potential caused by lamps with a solar emission spectrum or fluorescent tubes.

Reduced riboflavin acts as a catalyzer for the photochemical degradation of methionine, an amino acid contained in wine, when this is subjected to radiations comprised between 370 nm and 450 nm. From this degradation, as products of the reduction reaction, sulfate compounds belonging to the category of thiols or mercaptans, such as methanethiol, ethanol and methanol or dimethyl sulfate develop.

These compounds are foul smelling and have nauseating odors with low perception thresholds, which compromise the quality and modify the organoleptic properties of the wine in which they originate.

The onset of said unpleasant conditions is also known in the wine-making sector as "taste of light", since it typically derives from exposing the drink to luminous radiation.

Many analysis methods are known to analyze the properties of fluid products, particularly food products, in order to evaluate the stability of their duration if preserved in a warehouse or on the shelf. This is known in the field as shelf-life.

Such methods comprise, for example, oxidizing stability tests, which evaluate the tendency of the product to oxidize, protein stability tests, which evaluate the protein content by means of induced precipitation of proteins, and tartaric stability tests.

At present Applicant is not aware of standardized methods for determining, scientifically and repeatably, the stability of a fluid product exposed to light.

This is a disadvantage, if compared for example with the growing need on the part of producers to deal with complaints or returns from consumers who discover aroma and organoleptic anomalies in the food products they have purchased. Such anomalies, which in drinks typically consist of unpleasant sulfur smells due to the chemical alteration caused by prolonged exposure of the drinks to light, entail a need, for each drink or other fluid product that can be altered by luminous radiations, to detect and determine its stability to light before it is distributed.

It is also known that many fluid products are normally subjected, during their production process, to a series of treatments intended to obtain determinate chemical, physical or esthetic properties.

Among the treatments to which some fluid products are subjected, for example wines, there is a clarification treatment used to make them limpid. Typically, clarification provides to add substances called clarifiers or adjuvants to the wines and/or musts, which substances accelerate the sedimentation process, de-activating and adsorbing the substances that make the wine cloudy, and causing them to precipitate.

This treatment is particularly important in the case of bottling in transparent containers, through which the drink contained therein, and any possible cloudiness thereof, is clearly visible.

The fluid products are also subjected to other treatments, which normally provide to use technological additives or adjuvants.

The fluid products, especially if contained in transparent containers, and particularly if these are colorless, are more affected by exposure to light, which is not filtered (except minimally) by the container, and therefore they can be subject to alterations.

In the case of wines, such alterations mainly affect the organoleptic properties, and therefore they tend to take on the "taste of light" as described above.

With a view to optimizing production costs and times of a fluid food product, such as for example wine, clarifiers have been experimented with and embodied that allow both to make the fluid product limpid and also to reduce its riboflavin content, and hence possible sulfur derivatives, effectively contrasting the onset of the "taste of light".

Other technological additives or adjuvants can also be developed to this effect.

One purpose of the present invention is to obtain a device that allows to subject fluid products to luminous stress, effectively simulating various conditions of light exposure and which is able, at the same time, to keep the fluid products at optimum preservation temperatures, so as to evaluate the effect of the light alone on their chemical, physical and organoleptic characteristics and properties.

Another purpose of the present invention is to obtain a device to analyze fluid products that has considerable flexibility in use, that is inexpensive, portable, safe and easy to use, and that allows to analyze the fluid products both during the production process, and at the end of it, and also after distribution and sale.

Another purpose of the present invention is to perfect a method to analyze fluid products that allows to evaluate selectively, quickly and reliably the behavior of the products subjected to exposure to light, and also the efficacy of the technological additives or adjuvants used to improve their chemical, physical and organoleptic properties.

Another purpose of the present invention is to perfect a method to analyze fluid products that allows, in a uniform, standardized, repeatable and reliable manner, to determine the stability to light of the fluid products, obtaining data and results that are independent of the temperature and comparable between different fluid products.

Another purpose of the present invention is to obtain a kit that allows to subject fluid products to luminous stress and to analyze them so as to define preservation methods suitable to keep their chemical, physical and organoleptic properties unaltered.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a device according to the present invention to analyze fluid products is configured to subject fluid products to luminous stress, and comprises at least a containing body, such as a case, of the portable type and completely closable.

The containing body is hollow inside to define an internal compartment in which one or more receptacles containing the fluid products are completely closed.

Moreover, the containing body is defined by a first part or first shell, and by a second part or second shell, hinged to each other, and delimited by external walls formed by plates made of heat conductive material.

The device also comprises a lighting unit, provided with a plurality of sources of light and positioned inside the internal compartment, and a power unit associated to the containing body. The light sources are located in contact with one or more of the plates, which are configured to dissipate by conduction at least the heat produced by the sources of light toward the outside of the internal compartment.

The device to analyze fluid products also comprises, for each of the parts of the container, at least a transparent sheet made of methacrylate, or glass, or other similar materials, positioned substantially parallel to one of the plates, so that the sources of light are interposed between the plate and a corresponding transparent sheet. In particular each transparent sheet is configured to at least partly insulate thermally the sources of light from the internal compartment and from the one or more receptacles.

In this way there is the advantage of obtaining a compact and complete device, easy to transport, and able to passively dissipate at least part of the heat generated by the functioning of the lighting unit.

The device allows to subject the fluid products to luminous stress, by means of the lighting unit, without also inducing heat stress on them.

This allows to make the luminous stress treatments repeatable and make the analyses on the fluid products subjected to such treatments reliable.

According to one aspect of the present invention, the lighting unit is configured to selectively emit luminous radiations on selectively variable wavelengths.

In some forms of embodiment, there are seven selectively luminous radiations which can be used for the luminous stress, comprised in the emission spectrum of visible light and obtainable by a combination of three main radiations.

In possible implementations, the device also comprises one or more thermostating bodies at a lower temperature than the temperature of the fluid products, positioned inside the internal compartment in proximity to, or in contact with, the one or more receptacles and configured to maintain the temperature of the fluid products contained in the latter nearly constant.

In this way, a thermal barrier is advantageously obtained against the heating of the fluid products during the luminous stress treatment which is added to the passive dissipation performed by the plates mentioned above.

The present invention also concerns a method to analyze fluid products, and comprises:
- carrying out one or more improvement processes to improve the chemical-physical properties of the fluid products by means of one or more technological additives or adjuvants;
- inserting the fluid products in one or more receptacles;
- making available a device as shown above;
- inserting one or more receptacles and thermostating bodies in the internal compartment of the device in proximity to, or in contact with, the one or more receptacles;
- driving one or more of either a first switch, a second switch or a third switch, to selectively determine one of seven luminous radiations with a wavelength comprised between 350 nm and 700 nm;
- closing the device, so that the internal compartment is inaccessible from the outside, and subsequently activating an electric power supply to determine the switching on of sources of light present inside the internal compartment;
- after a treatment time comprised between about 5 minutes and about 12 hours, de-activating the electric power supply and removing the one or more receptacles from the internal compartment;
- analyzing the fluid products contained in the one or more receptacles to obtain data concerning the chemical, physical, microbiological and organoleptic properties of the fluid products;
- processing the data to determine the stability to the light, the optimal light for the preservation of the fluid products and the effectiveness of the one or more technological additives or adjuvants used in the initial step of the method.

The present invention advantageously allows to evaluate the effectiveness of the one or more improvement treatments and determine the most correct dosage of the one or more adjuvants and/or additives to confer resistance to luminous stress on each specific fluid product.

Moreover, the invention allows to evaluate the efficiency of the techniques that the whole productive process can improve the shelf life of the fluid products.

The method described above has the added advantage of allowing to attain a standard of analyses of fluid products, which is repeatable, easy to carry out and control, quick and reliable and therefore efficiently usable for the predetermined purposes mentioned above.

The present invention also concerns a kit to analyze fluid products comprising a device as indicated above, one or more receptacles to contain the fluid products, technological additives and adjuvants for treatments to improve the chemical-physical properties of the fluid products, one or more apparatuses for the chemical, physical and microbiological analysis of the fluid products, one or more tasting containers for the organoleptic analysis of the fluid products after the luminous stress treatment.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a three-dimensional view of a device according to the present invention;
- fig. 2 is a plan view of a part of the device in fig. 1;
- fig. 3 is a section view of the device in fig. 2.

### DETAILED DESCRIPTION OF SOME FORMS OF EMBODIMENT

With reference to the attached drawings, a device to subject fluid products to luminous stress is indicated in its entirety by the reference number 10 and comprises a containing body, in this case a case 11, containing a lighting unit 12 fed by a power unit 13.

In the present description reference will be made to "drinks", meaning that the considerations that will be made are also valid for intermediate semi-worked products in the production process.

This choice should only be taken as an example of a possible specific case of fluid product, and is not intended to limit the possible uses of the present invention to drinks alone. Indeed, the following description can be adapted to any other fluid product which is intended to be subjected to an evaluation of its stability to light.

By luminous stress we mean a treatment that provides to subject the drinks to luminous radiation emitted by the lighting unit 12 for a determinate treatment time.

The case 11 can be made of plates 14 of pure aluminum, or of a high degree of purity, connected to each other to each define a face or wall of an internally hollow parallelepiped.

In some forms of embodiment, such as those shown in the attached drawings, the plates 14 can be surrounded and connected to each other by perimeter section shapes 15.

The case 11 advantageously defines a portable containing body and is provided with an internal compartment 16 delimited by the plates 14.

In possible implementations, the case 11 is provided, on one of its sides, with a handle 18 attached to the corresponding plate 14 or to one of the perimeter section shapes 15.

The device 10 is thus compact and easy to transport, which also allows to carry out the luminous stress on the drinks directly at their production sites.

The device 10 can therefore be used to carry out luminous stress both on bottled drinks (for example in 750 ml bottles with a cork or screw top, or crown cap) or in an intermediate step of the production process, using the type of container considered most suitable to the purpose. The device 10 can therefore be used to verify the best way of preserving the drink in a new container (for example: a bottle in dark colored glass compared with one in clear glass, etc.).

The case 11 is also conventionally made in two parts, or shells, hinged with respect to each other that define a lid 11a, and a bottom 11b.

The lid 11a and the bottom 11b are typically configured to reciprocally rotate and to make the case 11 assume at least an open position and a closed position in the known way.

In the closed position, the lid 11a and the bottom 11b make the internal compartment 16 inaccessible from the outside.

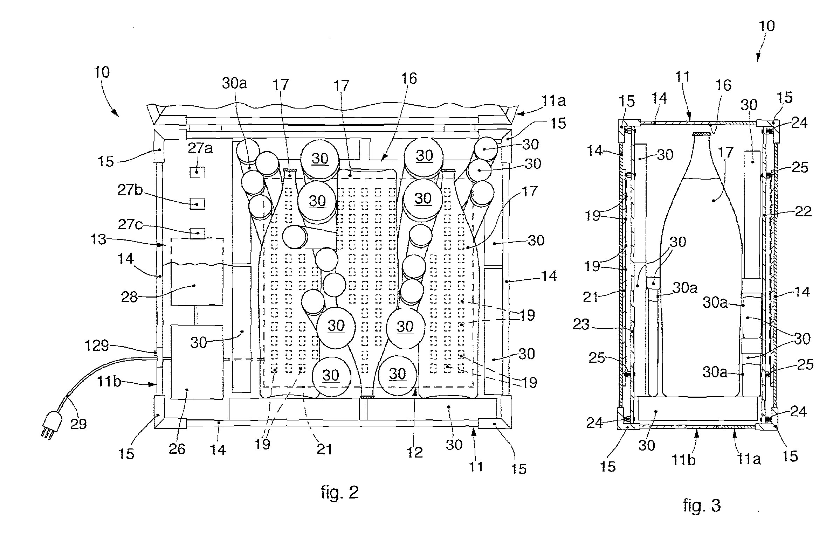

With reference to one use of the present device 10, that is, to subject drinks to luminous stress, fig. 2 shows how the open position allows to prepare and position the receptacles 17, such as bottles, test tubes or glasses, containing drinks to be treated, in the internal compartment 16. The closed position (fig. 3) on the other hand, both allows an easy transport of the case 11 and also defines the treatment position of the drinks.

The drinks are subjected to luminous stress with the case 11 in the closed position. This strategy makes the device 10 secure, since it prevents persons positioned near the case 11 from being dazzled by the light emitted by the lighting unit 12 during treatment.

In fig. 3 the case 11 is shown, merely for ease of illustration, resting on a smaller face, but this does not prevent the execution of the luminous stress even when the case rests on its larger face.

In some forms of embodiment, the lid 11a and the bottom 11b each define half of the case 11.

In other forms of embodiment, the lid 11a can define a part of the case 11 that is smaller in size than the bottom 11b.

In this way, the bottom 11b includes the largest part of the internal compartment 16 and is destined to contain the receptacles 17.

For example, the bottom can have sizes of about 460 mm x 360 mm x 80 mm, so as to be able to contain at least three standard 750 ml wine bottles.

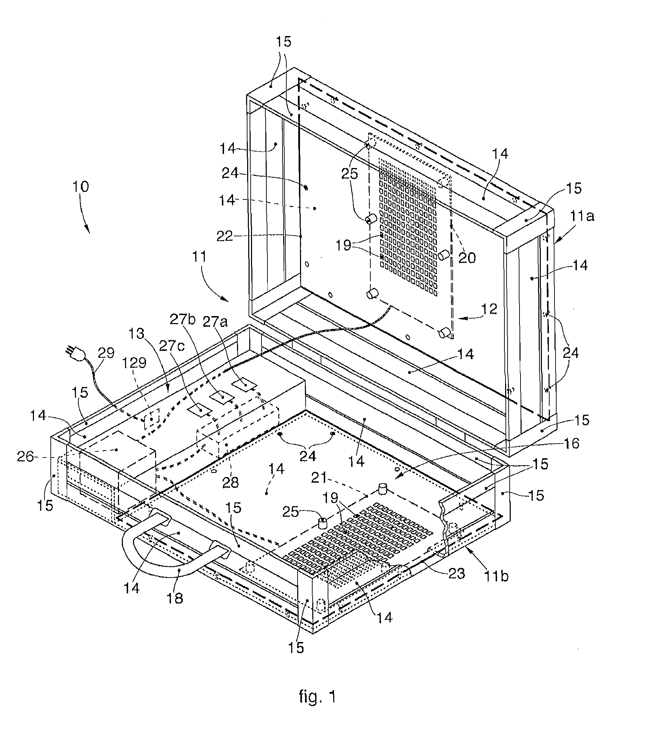

In some forms of embodiment, such as those shown by way of example in figs. 1 and 2, the lighting unit 12 and the power unit 13 can be positioned inside the case 11, in the internal compartment 16.

Other forms of embodiment can provide that the power unit 13 is in any case associated to the case 11 and positioned, wholly or in part, outside the case 11.

The power unit 13 can be connected or connectable to the lighting unit 12.

The use of pure aluminum is intended to confer high heat exchange capacities on the case 11 between the internal compartment 16 and the outside, so as to dissipate possible heat generated at least by the lighting unit 12 during functioning.

In the case of a power unit 13 inside the internal compartment 16, the heat possibly generated by the power unit 13 is also dissipated.

The lighting unit 12 can include one or more sources of light 19, which can be, as in the case shown here by way of example, Light Emitting Diodes or LEDs.

In some forms of embodiment, the sources of light 19 can be single or grouped in strips or sheets. The sources of light 19 can be ordered in rows or single files, or defining a polygonal or rectangular matrix disposition.

In fig. 1 an example of a lighting unit 12 is shown that includes a first sheet of LEDs 20, adhering to the internal surface of the upper plate 14 of the lid 11a, and a second sheet of LEDs 21, adhering to the internal surface of the lower plate 14 of the bottom 11b.

Each sheet of LEDs 20, 21 can, for example, contain about 200 to about 400 sources of light 19.

The adhesion of the sheets of LEDs 20, 21 to the corresponding plates 14 can be obtained by means of direct attachment thereto by attachment means such as screws, nails or rivets, or by adhesive paste.

Forms of embodiment are possible, such as those shown by way of example in the attached drawings, in which the device 10 includes a first transparent sheet 22 and a second transparent sheet 23, made of methacrylate for example, or glass, which determine the indirect attachment respectively of the first sheet of LEDs 20 and the second sheet of LEDs 21 to the case 11.

The first transparent sheet 22 and the second transparent sheet 23 can be, preferably, colorless.

Fig. 1 is used to describe forms of embodiment in which attachment members 24, such as screws, bolts, nails, rivets or similar, determine the attachment of the transparent plates 22, 23 to the perimeter section shapes 15, while anchoring elements 25, such as screws, bolts, nails, rivets or similar are configured to anchor each sheet of LEDs 20, 21 to one of the transparent sheets 22, 23.

The anchoring elements 25 are also configured to keep the first sheet of LEDs 20 and the second sheet of LEDs 21 adhering to the respective plates 14.

The purpose of this adhesion is to put the sheets of LEDs 20, 21 in contact with the plates 14, to allow the dissipation toward the outside of the case 11 of the heat that these can generate during use.

The greater the contact surface between the sheets of LEDs 20, 21 and the plates 14, the greater the heat dissipated by conduction during the luminous stress treatment.

Thanks to the material of which it is made, the case 11 acts as a passive dissipater of the heat generated by the lighting unit 12.

The presence of the transparent sheets 22, 23 determines a separation between the sheets of LEDs 20, 21 and the internal compartment 16, creating a partial insulation of the latter with respect to the heat generated by the sources of light 19 when they are activated.

This allows a greater efficiency of the device 10 in keeping the temperature of the drinks contained in the receptacles 17 inside the internal compartment 16 low.

Some solutions can provide to interpose, between each of the sheets of LEDs 20, 21 of the lighting unit 12 and the respective plate 14 of the case 11, a layer of conductive paste, or heat insulating gel, to increase the heat dissipation efficiency.

For example, a conductive paste containing metal particles with a high conductivity can be used, of silver or zinc or particles with a silicate base.

In possible implementations, both the first sheet of LEDs 20 and the second sheet of LEDs 21 can be of sizes such as to cover the whole surface of the respective plate 14.

Other implementations can provide that one of either the first sheet of LEDs 20 or the second sheet of LEDs 21 is smaller with respect to the surface of the corresponding plate 14.

It is also possible that both the first sheet of LEDs 20 and the second sheet of LEDs 21 are smaller with respect to the surfaces of the corresponding aluminum plates 14.

In such solutions, the first sheet of LEDs 20 and the second sheet of LEDs 21 can be partly or completely overlapping when the case 11 is in the closed position.

The power unit 13 includes an electric power supply 26, to which both the first sheet of LEDs 20 and the second sheet of LEDs 21 are connected and from which they receive the necessary electric power to activate all the sources of light 19.

The electric power supply 26 can be activated either directly through the connection of an electric cable 29 to the power distribution network, or through said connection and subsequent drive of an external switch 129 positioned on one side of the case 11 and connected to the electric power supply 26.

The power unit 13 also includes a first switch 27a, a second switch 27b and a third switch 27c, all three connected to a switching unit 28, connected in its turn to the electric power supply 26.

The connection circuit that connects the switches 27a, 27b, 27c, switching unit 28 and electric power supply 26 is such that driving the first switch 27a determines the emission of red light from the sources of light 19 of the sheets of LEDs 20, 21.

Similarly, driving the second switch 27b determines the emission of green light, while driving the third switch 27c determines the emission of blue light.

The colors, red, green or blue constitute the trio of main colors of the additive model "RGB".

The luminous radiation of red emitted by the sources of light 19 when only the first switch 27a is driven has a wavelength comprised between about 610 nm and about 700 nm, for example between 620 nm and 630 nm.

The luminous radiation of green emitted by the sources of light 19 when only the second switch 27b is driven has a wavelength comprised between about 510 nm and about 560 nm, for example between 520 nm and 535nm.

The luminous radiation of blue emitted by the sources of light 19 when only the third switch 27c is driven has a wavelength comprised between about 450 nm and about 500 nm, for example between 460 nm and 475 nm.

The three switches 27a, 27b, 27c can be driven either singly, to obtain the three main colors, or in any of their combinations, to obtain secondary colors.

The switching unit 28 is able to manage the electric drive signals coming from the switches 27a, 27b, 27c in order to combine the main colors and obtain another four secondary colors. There are thus seven colors of light obtainable and usable to subject the drinks contained in the receptacles 17 to luminous stress.

Indeed, using the additive method RGB, the secondary colors obtainable with pairs of main colors are yellow, cyan and magenta.

When all three switches 27a, 27b, 27c are switched on at the same time, the sources of light 19 of the first sheet of LEDs 20 and of the second sheet of LEDs 21 emit white light.

The luminous radiations obtainable have wavelengths comprised between about 350 nm and about 700 nm, that is, they cover the field of visible light almost completely.

In this way the advantage is obtained of being able to selectively decide which of the seven luminous radiations to use for the treatment of alcoholic drinks.

In figs. 1 and 2 an electric cable 29 is schematically shown, which establishes the connection of the electric power supply 26 to the network.

Fig. 2 is used to describe possible forms of embodiment of the device 10 in which it includes a series of thermostating bodies 30, positioned in the internal compartment 16 and configured to maintain nearly constant the temperature of the drinks contained in the receptacles 17.

A slight overheating of the drink, in the order of a few degrees for example, can however be acceptable, since it does not cause physical-chemical alterations such as protein "casse", that is, the precipitation of the proteins due to excessive heating of the drink that contains them.

The thermostating bodies 30 can each be defined by a container 30a, with a hermetic seal, at least partly filled with a fluid or with a eutectic mix with a high specific heat. An example of thermostating body 30 can be a eutectic plate of the known type, while an example of a fluid with a high specific heat can be pure propylene glycol, and an example of eutectic mix can be a mixture of propylene or ethylene glycol and water in a 20% solution.

The use of propylene glycol, whether pure or mixed with water, is advantageous in that this fluid is colorless, and therefore does not impede, deviate or absorb luminous radiations emitted by the sources of light 19, thus it does not affect the quantity of light that reaches the drinks to be treated.

Other fluids or mixtures transparent to the luminous radiations comprised in the spectrum of visible light, and preferably colorless, can be used as an alternative to propylene or ethylene glycol.

The thermostating bodies 30 are introduced into the internal compartment 16 at a temperature lower by even twenty degrees than the temperature of the alcoholic drinks contained in the receptacles 17. Since they are configured to have high heat inertia, the thermostating bodies 30 act as a heat barrier against the heat generated by the lighting unit 12 and by the power unit 13 when the sources of light 19 are activated and the luminous stress of the drinks is in action.

Consequently, while the plates 14 and therefore the walls of the case 11 in general act as passive dispersers of the heat toward the outside of the internal compartment 16, the thermostating bodies 30 counterbalance said heat with their own heat inertia, preventing this from influencing the temperature of the drinks even under the most severe conditions of use.

In this way, it is advantageously obtained that the drinks, when they are subjected to luminous stress, maintain an almost constant temperature, or one that varies only by a few degrees. If, after this treatment, the drinks undergo chemical, physical or organoleptic modifications or alterations, these are attributable only to the light, and not also or alternatively to the temperature.

The possibility that determinate wavelengths can selectively be set, and the fact that possible alterations of the drinks are not caused by the temperature, due to passive dissipation and heat barrier, allows the device 10 to carry out repeatable treatments, which are also efficient in determining the effect of the light on alcoholic drinks.

In particular, the device 10 as described above can be used to carry out an essential step in a method to analyze the stability to light of drinks or other fluid products.

The method can provide an initial step of preparing the drink or drinks to be analyzed. The initial step can include treatments to improve the chemical-physical properties of the drinks and can be carried out using technological additives and/or adjuvants during the entire productive process. By improving treatment we mean an operation to modify the chemical or physical conditions of the drinks, or semi-worked products, for example by adding substances which determine pre-determined chemical-physical conditions.

The initial step can include, for example, clarification of the drinks or the intermediate semi-worked products in the production process, such as musts for wines, for example.

Clarification occurs by adding a determinate amount of clarifying agent, which in wines can be comprised for example between about 5 g/hl and 150 g/hl, and subsequent filtering to eliminate the clarification dregs or sedimentation.

Once clarified, or in any case subjected to improvement treatments, the alcoholic drinks are inserted into receptacles 17, such as 750 ml or 350 ml bottles, or 40 ml test tubes, or even in beaker test tubes for laboratory analyses, or other receptacles suitable to the purpose.

At this point, the analysis method can provide a step of cooling the receptacles 17 containing the drinks to an optimal preservation temperature. The optimal preservation temperature depends on the drink, and for wines it is comprised between about 10°C and 15°C. For some drinks the preservation temperature and the temperature to serve to the final client can be little more than 0°C.

Subsequently, the treatment device 10 is prepared and the case 11 is put in its open condition, so as to be able to insert the receptacles 17 in the internal compartment of the case 11.

In possible implementations, in the internal compartment 16, between the receptacles 17, the thermostating bodies 30 are interposed that have been previously cooled to a temperature lower than the temperature of the receptacles 17.

The luminous radiation to be used is then selected by driving one or more of the first 27a, second 27b, third 27c switches.

Afterward, the case 11 is put in its closed condition, rendering the internal compartment 16 inaccessible from the outside, and activating the electric power supply 26, either by connecting the electric cable 29 to the power distribution network or by means of said connection and driving the external switch 129.

This activation determines the switching on of the sources of light 19 and in practice the beginning of the luminous stress.

One possible implementation of the method can provide that the treatment time is controlled by means of a timer, that is, the time the drinks are exposed to the light.

This exposure can be comprised between a few minutes, 5 to 10 for example, up to a few hours, 12 for example.

After the treatment time has passed, the electric power supply 26 is deactivated, the case 11 is opened and the receptacles 17 extracted from the internal compartment 16.

The method under discussion provides to carry out, using known apparatuses, chemical, physical and microbiological analyses and organoleptic tests on drinks once the receptacles 17 have been removed from the internal compartment 16.

The organoleptic tests can provide that the drinks are transposed from the receptacles 17 into one or more tasting glasses.

From these analyses data can thus be deduced concerning the presence and the entity of possible chemical, physical, microbiological or organoleptic alterations of the drinks.

A final step of processing the data cited above allows to determine the stability to the light of the drinks treated, in particular which luminous radiation most affects its properties and which is the exposure time which can give rise to alterations.

This processing step can include an interpolation of the data that allows to use a luminous stress carried out for 10 minutes on 40 ml of product to simulate the exposure of the drink to a year of luminous radiation, and therefore to determine the stability to light of the drink itself.

It is clear that modifications and/or additions of parts may be made to the device and method to analyze fluid products as described heretofore, without departing from the field and scope of the present invention.

It is also clear that, although the present invention has been described with reference to some specific examples, a person of skill in the art shall certainly be able to achieve many other equivalent forms of device and method, having the characteristics as set forth in the claims and hence all coming within the field of protection defined thereby.

## Claims

1. Device to analyze fluid products, configured to subject fluid products to luminous stress, **characterized in that** it comprises:
- a containing body (11) hollow inside to define an internal compartment (16) for housing one or more receptacles (17) containing said fluid products, said containing body (11) being the portable type and completely closable to completely close inside it said one or more receptacles (17), said containing body (11) being defined by a first part or first shell (11a), and by a second part or second shell (11b), hinged to each other, said first part or first shell (11a) and said second part or second shell (11b) being delimited by external walls formed by plates (14) made of heat conductive material,
- a lighting unit (12), positioned inside said internal compartment (16), provided with a plurality of sources of light (19), and a power unit (13) associated with said containing body (11),
wherein said sources of light (19) are put in contact with one or more of said plates (14), said plates (14) being configured to dissipate by conduction toward the outside of the internal compartment (16) at least the heat produced by the sources of light (19), said device to analyze fluid products also comprising, for each of said parts (11a, 11b) of the containing body (11), at least a transparent sheet (22, 23), positioned substantially parallel to one of said plates (14), said sources of light (19) being interposed between the plate (14) and a corresponding transparent sheet (22, 23), each transparent sheet (22, 23) being configured to at least partly insulate thermally the sources of light (19) from the internal compartment (16) and from the one or more receptacles (17).

2. Device as in claim 1, **characterized in that** said lighting unit (12) is configured to selectively emit luminous radiations on selectively variable wavelengths.

3. Device as in claim 1 or 2, **characterized in that** said plates (14) are made of a material chosen from a group comprising at least pure aluminum, aluminum alloys, pure copper, alloys of copper and silver.

4. Device as in any claim hereinbefore, **characterized in that** said power unit (13) is connected or connectable to said lighting unit (12) and is configured to electrically feed said lighting unit (12).

5. Device as in any claim hereinbefore, **characterized in that** said power unit (13) comprises an electric power supply (26) connected to the sources of light (19) of said lighting unit (12) and to a switching unit (28), a first switch (27a), a second switch (27b) and a third switch (27c) connected to said switching unit (28) and configured to determine the emission by said sources of light (19) of luminous radiations having wavelengths comprised respectively between about 610 nm and 700 nm, between about 510 nm and about 560 nm, and between about 450 nm and about 500 nm.

6. Device as in claim 5, **characterized in that** said first (27a), second (27b) and third (27c) switch can be driven singly or simultaneously, in a plurality of combinations, **and in that** said switching unit (28) is configured to determine the emission by said sources of light (19) of luminous radiations of at least seven distinct colors in the field of visible light.

7. Device as in any claim hereinbefore, **characterized in that** it comprises one or more thermostating bodies (30) at a lower temperature than the temperature of said fluid products, positioned inside said internal compartment (16) in proximity to, or in contact with, said one or more receptacles (17) and configured to maintain nearly constant the temperature of the fluid products contained in said receptacles (17).

8. Method to analyze fluid products, **characterized in that** it comprises:
- carrying out one or more improvement processes to improve the chemical-physical properties of the fluid products by means of one or more technological additives or adjuvants;
- inserting said fluid products in one or more receptacles (17);
- making available a device (10) as in any claim from 1 to 7;
- inserting said one or more receptacles (17) and thermostating bodies (30) in the internal compartment (16) of the device (10) in proximity to, or in contact with, said one or more receptacles (17);
- driving one or more of either a first switch (27a), a second switch (27b) or a third switch (27c), to selectively determine one of seven luminous radiations with a wavelength comprised between 350 nm and 700 nm;
- closing said device (10), so that said internal compartment (16) is inaccessible from the outside, and subsequently activating an electric power supply (26) to determine the switching on of sources of light (19) present inside said internal compartment (16);
- after a treatment time comprised between about 5 minutes and about 12 hours, de-activating the electric power supply (26) and removing said one or more receptacles (17) from said internal compartment (16);
- analyzing the fluid products contained in said one or more receptacles (17) to obtain data concerning the chemical, physical, microbiological and organoleptic properties of said fluid products;
- processing said data to determine the stability to light, the optimal light for the preservation of said fluid products and the effectiveness of said technological additives or adjuvants.

9. Method as in claim 8, **characterized in that,** before inserting the thermostating bodies (30) in said internal compartment (16), it provides to cool them to a temperature lower by at least 10°C than that of said one or more receptacles (17).

10. Kit to analyze fluid products comprising a device (10) as in any claim from 1 to 7, one or more receptacles (17) to contain said fluid products, additives and technological adjuvants to improve the chemical-physical properties of said fluid products, one or more apparatuses for the chemical, physical and microbiological analysis of said fluid products, one or more tasting containers for the organoleptic analysis of said fluid products.
